# EUROPÄISCHE PATENTSCHRIFT

(11) **EP 1 392 369 B1**
(45) Veröffentlichungstag und Bekanntmachung des Hinweises auf die Patenterteilung: **19.11.2008**
(21) Anmeldenummer: 02743037.0
(22) Anmeldetag: 16.05.2002
(51) Int. Cl.: A61L 15/00, A61K 36/03, A61K 36/04, A61K 36/05

(54) **VERWENDUNG VON BLÄTTERN ZUR HERSTELLUNG VON KÖRPERUMSCHLÄGEN SOWIE VORPRODUKT UND VORRICHTUNG DAFÜR**
USE OF SHEETS FOR PRODUCING BODY COMPRESSES AND A PRE-PRODUCT AND DEVICE THEREFOR
UTILISATION DE FEUILLES POUR PRODUIRE DES COMPRESSES CORPORELLES, PRODUIT SEMI-FINI ET DISPOSITIF A CET EFFET

(30) Priorität: 31.05.2001 DE 10126577
(43) Veröffentlichungstag der Anmeldung: 03.03.2004
(73) Patentinhaber: Haslauer, Paul, A-5020 Salzburg (AT)
(72) Erfinder: Haslauer, Paul, A-5020 Salzburg (AT)
(74) Vertreter: Flach, Dieter Rolf Paul
(86) Internationale Anmeldenummer: PCT/EP2002/005422
(87) Internationale Veröffentlichungsnummer: WO 2002/096498

(56) Entgegenhaltungen:
- DE-A- 2 625 643
- FR-A- 2 581 666
- DATABASE WPI Section Ch, Week 198943 Derwent Publications Ltd., London, GB; Class A96, AN 1989-312264 XP002212236 & JP 01 228916 A (KITAMURA K), 12. September 1989 (1989-09-12)
- DATABASE WPI Section Ch, Week 199837 Derwent Publications Ltd., London, GB; Class B07, AN 1998-431460 XP002212237 & JP 10 179764 A (KYOWA KK), 7. Juli 1998 (1998-07-07)
- DATABASE WPI Section Ch, Week 199805 Derwent Publications Ltd., London, GB; Class B04, AN 1998-046913 XP002212238 & JP 09 295934 A (NAGAOKA JITSUGYO KK), 18. November 1997 (1997-11-18)
- DATABASE WPI Section Ch, Week 200014 Derwent Publications Ltd., London, GB; Class D21, AN 2000-154526 XP002212239 & JP 2000 014587 A (MYOJO SANSHO KK), 18. Januar 2000 (2000-01-18)
- PATENT ABSTRACTS OF JAPAN vol. 2000, no. 19, 5. Juni 2001 (2001-06-05) & JP 2001 039888 A (KAWAGOE YOSHIHARU), 13. Februar 2001 (2001-02-13)
- DATABASE WPI Section Ch, Week 198521 Derwent Publications Ltd., London, GB; Class D21, AN 1985-126094 XP002212240 & JP 60 064906 A (ERIMO-CHO), 13. April 1985 (1985-04-13)
- PATENT ABSTRACTS OF JAPAN vol. 005, no. 020 (C-042), 6. Februar 1981 (1981-02-06) & JP 55 145614 A (OKUYAMA MUNEAKI), 13. November 1980 (1980-11-13)
- DATABASE WPI Section Ch, Week 199613 Derwent Publications Ltd., London, GB; Class D15, AN 1996-127938 XP002212920 & SE 9 401 516 A (KLINGSTROM T V), 4. November 1995 (1995-11-04)
- DATABASE WPI Section Ch, Week 199241 Derwent Publications Ltd., London, GB; Class D13, AN 1992-338362 XP002212241 & KR 9 104 347 B (LEE S), 26. Juni 1991 (1991-06-26)
- DATABASE WPI Section Ch, Week 199610 Derwent Publications Ltd., London, GB; Class B05, AN 1996-095842 XP002213231 & NZ 244 678 A (STAPLETON W E), 21. Dezember 1995 (1995-12-21)
- DATABASE WPI Section Ch, Week 198613 Derwent Publications Ltd., London, GB; Class A96, AN 1986-084413 XP002212243 & JP 61 030517 A (NICHIBAN KK), 12. Februar 1986 (1986-02-12)

## Beschreibung

Die vorliegende Erfindung betrifft die Verbesserung des Wohlbefindens, Aussehens und des Gesundheitszustands von Menschen unter gezielter Nutzung und Beeinflussung von Stoffaustauschvorgängen, an denen die menschliche Haut beteiligt ist. Derartige Stoffaustauschvorgänge spielen insbesondere im Kur- und Badebereich eine wichtige Rolle, indem man mit die Gesundheit und das Wohlbefinden fördernden Bädern, Schlammpackungen und ähnlichem die über die Haut ablaufenden Stoffaustauschvorgänge nutzt und beeinflusst.

Es ist bekannt, dass die menschliche Haut ein Organ ist, das zahlreiche Aufgaben erfüllt. Zu diesen gehören insbesondere Schutzfunktionen, indem die Haut den Menschen einerseits vor einem Verlust lebenswichtiger Substanzen und Flüssigkeiten schützt, andererseits auch einen Schutz gegen das Eindringen schädlicher Substanzen und Mikroorganismen von außen bietet. Zusätzlich ist die Haut ist auch noch ein wichtiges Ausscheidungs- und Atmungsorgan.

Eine gesunde Haut ist in Hinblick auf die aus dem Körperinneren durch die Haut ausgeschiedenen Substanzen genauso selektiv wie im Hinblick auf die Substanzen, die von außen in die Haut eindringen können oder durch diese hindurch in den Kreislauf gelangen können. Es ist ein Kennzeichen vieler Hauterkrankungen, dass sie von Störungen der über die Haut ablaufenden Stoffaustauschvorgänge begleitet sind, und zwar entweder im Sinne einer Störung der Ausscheidungsfunktion und/oder der Schutzfunktion, die in das Eindringen körperfremder Substanzen in die Haut erschwert.

Beim über die Haut ablaufenden Stoffaustausch sind somit grundsätzlich zwei Richtungen und Vorgänge zu unterscheiden: ein von Innen nach Außen fließender Strom transportiert verschiedene Körperflüssigkeiten wie Wasser, Schweiß, Talg aus subkutanen Bereichen des menschlichen Körpers nach außen. Dieser Transport dient einerseits der Entfernung von Schlacken und Abfallstoffen des Körpers, andererseits erfüllen diese nach außen auf die Hautoberfläche transportierten Körpersubstanzen auch wichtige Funktionen. So wird auf der Haut aus den aus dem Körper hinaustransportierten Lipiden, Harnstoff sowie sauren Schweißbestandteilen ein wirksamer Schutzfilm gebildet, der tiefere Hautschichten gegen ein Eindringen schädlicher Bakterien und Viren schützt. Bei hohen Temperaturen bewirkt der Stofftransport von innen nach außen als Schwitzen auch eine Abkühlung des Körpers. Beim Schwitzen, oder wenn man den Körper bei Entschlackungskuren gezielt veranlasst, die Stoffausscheidung über die Haut zu erhöhen, werden jedoch nicht nur Schlackstoffe ausgelagert, sondern der Körper verliert auch wichtige Substanzen wie Vitamine, Mineralstoffe, Harnstoff und andere, die im Körper wichtige Funktionen erfüllen.

Was den Stofftransport von außen nach innen angeht, stellt die Haut eine wirksame mechanisch-chemische Barriere dar, die dann, wenn man beabsichtigt, der Haut oder dem Körper nützliche Stoffe zuzuführen, überwunden werden muss.

Die Beeinflussung des Stoffaustauschs über die Haut ist Teil der ältesten medizinischen Praktiken der Menschheit. Heilerden dienten dazu, durch Förderung des Stoffaustauschs von innen nach außen und Bindung von Schad- und Schlackstoffen den Gesundheitszustand von Patienten zu verbessern. Andererseits gehört es ebenfalls zu den ältesten medizinischen Praktiken der Menschheit, gesundheitsfördernde Stoffe durch die Haut zuzuführen, beispielsweise als Salben, Umschläge mit Pflanzenprodukten, z.B. Senfpflaster, oder Bäder unter Verwendung von Heilpflanzen und Heilpflanzenextrakten.

Die Zufuhr von medizinischen Wirkstoffen durch in die Haut stellt heute ein eigenes Sachgebiet der Pharmakologie dar. So werden zur Arzneimittelverabreichung transdermale Verabreichungssysteme eingesetzt, mit deren Hilfe Wirkstoffe verzögert über einen längeren Zeitraum an die Haut abgegeben und letztlich zur Absorption und damit zu einer systemischen Wirkung gebracht werden können. Der Vorgang des Eindringens von Stoffen in die Haut und das Vordringen eines Stoffes in tiefere Schichten wird auch als Permeation oder Absorption bezeichnet. Beim Eindringen von Stoffen lassen sich die aufeinanderfolgenden Schritte der Penetration, d.h. des Eindringens des Stoffs in die Haut, die Permeation, d.h. das Durchwandern der einzelnen Hautschichten, und die Resorption unterscheiden, die die Aufnahme des Stoffs in die Kapillaren beschreibt. Damit ein Wirkstoff vom Körper absorbiert wird, müssen alle drei Stufen durchlaufen werden. Es wurden bereits umfangreiche Untersuchungen mit zahlreichen Stoffen und Stoffgruppen durchgeführt, um deren Einfluss auf die Permeation festzustellen und ggf. eine permeationsbeschleunigende Wirkung nutzen zu können. Derartige Permeationsbeschleuniger sind wichtige Bestandteile von transdermalen Arzneimittelpflastern.

Eine Verbesserung der Permeation der Haut wird auch mit Hilfe der sog. Okklusionstechnik (Harris; Ziegenmeyer) erreicht. Die Okklusionstechnik wird typischerweise so angewandt, dass ein die zu verabreichenden Wirkstoffe enthaltendes Externum auf die Haut aufgetragen wird. Die Schicht wird dann mit einer dünnen, elastischen Spezialfolie abgedeckt und möglichst gleichmäßig flächig an den Körper angedrückt. Die Folie bewirkt, dass die durch die Haut nach außen abgegebenen Substanzen nicht verlorengehen können, sondern nur in die direkt über der Haut angeordnete Schicht des Externums gelangen, von wo aus sie in einem dynamischen Gleichgewicht wieder in die Haut zurückwandern können. Es kommt unter der geschlossenen Außenschicht der Okklusivfolie auf diese Weise zu einem intensivierten Stoffaustausch, der auch den Transport von Wirkstoffen in die Haut und durch diese hindurch fördert und zu diesem Zwecke gezielt genutzt wird. Es hat sich gezeigt, dass viele medizinische Wirkstoffe mittels einer transdermalen Verabreichung genau, schonend und mit hoher Konstanz an den Körper verabreicht werden können.

Angesichts des erhöhten transdermalen Stoffaustausches versteht es sich, dass die bei der Okklusionstechnik verwendeten Folien hohen Reinheitsanforderungen genügen müssen und beispielsweise keine schädlichen Stoffe wie Weichmacher an das Externum abgeben dürfen.

Zu den als "Externa" bezeichenbaren Substanzen, die wertvolle Inhaltsstoffe und Heilstoffe enthalten und auf die Haut aufgebracht werden, gehören auch zahlreiche Materialien auf natürlicher bzw. pflanzlicher Grundlage. Zu diesen Materialien gehören u.a. Kurpräparate, die aus Algen und dabei insbesondere aus den als Tange bezeichneten, blattartige Strukturen bildenden mehrzelligen Formen von Meeresalgen gewonnen werden.

Database WPI AN 1989-312264 bzw. JP 01 228 916 A beschreibt einen vorfabrizierten Beutel aus textilen Materialen oder Kunststofffolien, der getrockneten und zerkleinerten Seetang (0,1 mm bis 10 mm) enthält und als heiße Kompresse in der Unterwäsche zu tragen ist.

Database WPI AN 1998-431460 bzw. JP 10 179764 A beschreibt ein Pflaster, das eine Schicht einer Mischung von Mineralpulvern enthält, von denen eines eine Asche aus einem Tang sein kann.

Database WPI AN 1998-046913 bzw. JP 09 295934 A beschreibt ein Schönheitspflaster, das u.a. Extrakte aus Tangen enthalten kann.

Database WPI AN 2000-15426 bzw. JP 2000 014587 A beschreibt ein Papier oder Vlies für kosmetische Umschläge, das mit einer Salzmischung getränkt ist, die u.a. Asche aus der Tangverkohlung umfasst.

Patent Abstracts of Japan, Vol. 2000, No. 19 bzw. JP 2001 039888 A beschreibt ein netzartiges Tissuematerial, das mit Pulvern von essbaren Pflanzen beladen ist, die alle zu den Landpflanzen gehören.

Database WPI AN 1985-126094 bzw. JP 60 064906 A beschreibt die Herstellung eines pastenartigen Produkts, das durch Vermahlen von angefeuchteten UV-bestrahlten und getrockneten Tangen zu einem Pulver erhalten wird, das als Basis für kosmetische Produkte verwendet werden kann.

Patent Abstracts of Japan Vol. 005, No. 020 (C-042) bzw. JP 55 145614 A beschreibt die Bestrahlung von Flüssigkeiten mit sichtbarem Licht zur Erzielung einer Heilwirkung.

Database WPI AN 1996-127938 bzw. SE 9 401 516 A beschreibt ein Verfahren zur Abwasserbehandlung.

DE 26 25 643 A beschreibt Produkte sehr unterschiedlicher Art zur physikalisch-chemischen Aktivierung von Wasser, das aus Berieselungsdüsen, z.B. Duschen oder Wasserhähnen, austritt.

Database WPI AN 1992-338362 bzw. KR 9 104 347 B beschreibt ein Verfahren zum Trocknen von essbarem Tang in einem Heißlufttrockner und das Klassieren des Produkts.

Database WPI AN 1996-095842 bzw. NZ 244 678 A beschreibt eine kühlenden Salbe zur Behandlung von Verletzung des weichen Gewebes von Tieren, vor allem jungen Pferden, die u.a. zerkleinerten Seetang enthält.

Database WPI AN 1986-084413 bzw. JP 61 030517 A beschreibt einen an der Schleimhaut haftenden Verband, der ein Arzneimittel abgibt.

FR 2 581 666 A beschreibt eine gitterartige Kunststoff-, insbesondere Polypropylen-Folie für Kompressen.

Es ist Aufgabe der vorliegenden Erfindung, insbesondere bei Anwendungen im Kur- und Pflegebereich unter Verwendung von Materialien auf natürlicher Basis eine Verbesserung des Stoffaustauschs über die menschliche Haut zu erreichen und damit Kur- und Behandlungserfolge zu verbessern.

Diese Aufgabe wird dadurch gelöst, dass man zur Herstellung von Körperumschlägen für die kosmetische oder medizinisch-dermatologische Behandlung von Kurgästen und Patienten austauschaktive Blätter von Tangen gemäß Anspruch 1 verwendet.

Als "austauschaktive Blätter" werden dabei ganze Blätter oder Blatteile, bei denen wesentliche Flächenbereiche der Blattspreite ganzer Blätter erhalten sind, bezeichnet, bei denen ein intensiver Stoffaustausch durch die Blattoberfläche erfolgt. Als "Blätter" im Sinne dieser Erfindung sind somit z.B. auch von groben Blattrippen, -nerven oder -adern befreite flächige Blatteile und/oder durch furnierartiges Abschälen großer Blätter erhaltene Blattprodukte anzusehen. Besonders aktive "austauschaktive" Blätter sind dabei Blätter solcher Pflanzen, die Nährstoffe und andere Stoffe weniger über die Wurzeln aufnehmen, als direkt durch die Blätter. Zu den Pflanzen, die Stoffe aus ihrer Umgebung über die Blätter aufnehmen, gehören insbesondere im Wasser lebende Algen und Wasserpflanzen. Blattstrukturen bildende Algen gehören in erster Linie zu den Braunalgen, es gibt jedoch auch Blattstrukturen ausbildende Rot- und Grünalgen. Andere Pflanzen, für die bekannt ist, dass bei ihnen ein intensiver Stoffaustausch über Blätter und Blattstrukturen erfolgt, sind beispielsweise Farne.

Die Eigenschaft von Algen, in ihren als "Blätter" bezeichneten Organen selektiv die verschiedensten Stoffe aus dem Meer anzureichern, wird bereits vielfältig genutzt. So ist beispielsweise bekannt, dass einige Algenarten große Mengen an Jod anreichern. Dieses Jod sowie andere in Meeresalgen angereicherten Stoffe werden beispielsweise dadurch genutzt, dass Algen oder daraus erhaltene Aschen als wertvolle Dünger verwendet werden. Algen, insbesondere Braunalgen, enthalten jedoch auch medizinisch wirksame und/oder hautpflegende Inhaltsstoffe, weshalb Produkte auf Algenbasis auch in der Medizin und Kosmetik verwendet werden. Eine bekannte Möglichkeit, die aufgrund ihres intensiven Stoffaustauschs in Algen angereicherten Wirkstoffe zu nutzen, besteht darin, aus getrockneten und vermahlenen Algen mit Wasser einen Brei zu bereiten, der als dünne Schicht oder Packung (Externum) auf die Haut aufgebracht wird. Verwendet man Algen mit hohem Jodgehalt wie beispielsweise Algen der Art Laminaria digitata (Fingeralgen), können diese Jodgehalte in gesundheitsfördernder Weise genutzt werden.

Der vorliegenden Erfindung liegt die Erkenntnis zugrunde, dass in einem solchen Falle sowie in ähnlichen Fällen der Stoffaustausch mit den als Externa auf die Haut aufgebrachten Substanzen erhöht werden kann, wenn man zusätzlich Blätter oder Blatteile von Algen als äußere Schicht über einem Externum aufbringt. Wenn man beispielsweise auf eine Schicht aus zu einem Pulver vermahlenen und zu einem pastösen Brei oder Gel verarbeiteten Laminaria digitata Algen, die einen hohen Jodgehalt von ca. 420 mg/kg aufweisen, Blätter einer anderen Algenart, die selbst nur einen geringen Jodgehalt aufweisen kann, aufbringt und diese Algenblätter als äußere Schicht in der Art einer Okklusionsfolie verwendet, beobachtet man einen erhöhten Stoffaustausch. Die austauschaktiven Blätter treten in eine Wechselwirkung mit dem Externum, indem sie die vom Körper in dieses abgebenen Stoffe reversibel aufnehmen und permeationsfördernde Inhaltsstoffe reversibel in das Externum abgeben und dadurch im Sinne eines externen Ausgleichsspeichers ausgleichend und begünstigend auf den gewünschten Stoffaustausch Einfluss nehmen.

Die Austauschleistung und die Fähigkeit der Blätter, den Stoffaustausch zu fördern, können ggf. noch dadurch verbessert werden, dass man die Blätter bzw. den Körperumschlag mit der Außenschicht aus austauschaktiven Blättern einer Einwirkung von Licht aussetzt, so dass die revitalisierten Blätter auch ihre Photosynthese-Leistung aufnehmen und auch in diesen Sinne zusätzlich aktiviert werden.

Es hat sich ferner gezeigt, dass der permeationsfördernde Effekt von austauschaktiven Blättern noch verstärkt wird, wenn man diese Algen auf einem Externum oder direkt auf der Haut unter einer üblichen, für die Okklusionstechnik verwendeten Folie einsetzt. Verschiedene Algen, die z.B. jodarm sein können, jedoch auch Iodgehalte im Bereich des Iodgehalts des Externums oder darüber aufweisen können, eignen sich auch deshalb für die genannte Anwendung sehr gut, weil sie relativ großflächige Blätter aufweisen und auch dann, wenn sie vorher getrocknet waren, nach dem Einweichen sehr weich und geschmeidig werden und daher für die Haut sehr angenehm sind. Gemäß einer bevorzugten Ausführungsform werden die austauschaktiven Blätter somit so zur Herstellung von Körperumschlägen verwendet, dass man zuerst ein pastöses wirkstoffreiches Externum aus gemahlenem jodreichen Braunalgen aufbringt und darauf eine Schicht von Blättern von weichen Algen, die einerseits permeationsfördernd sind, andererseits Stoffverluste begrenzen. Die Wirkung kann durch eine zusätzliche externe Okklusionsfolie noch verstärkt werden.

Es hat sich als praktisch erwiesen, die zur Herstellung der Körperumschläge vorgesehenen Algen als spezielle Vorprodukte bereitzustellen, bei denen schichtartig ausgebreitete austauschaktive Blätter in trockener oder feuchter Form auf ein Bahnmaterial aufgelegt sind, das vlies-, gaze- oder netzartig ist und sowohl aus feuchtigkeitsabweisenden als auch aus feuchtigkeitsaufnehmenden Materialen hergestellt sein kann. Dieses Bahnmaterial kann mit der daraufliegenden Blätterschicht beispielsweise zu einer Rolle gerollt werden, das Bahnmaterial kann jedoch auch in einzelne Abschnitte geschnitten und in der Art von Papierhandtüchern und Servietten als Faltstapel vorliegen. In jedem Falle soll beim Abziehen des Bahnmaterials von der Rolle oder aus dem Faltstapel eine gleichmäßige Schicht an Blättern auf dem feuchtigkeitsdurchlässigen Bahnmaterial-Träger erhalten werden. Das Bahnmaterial mit den darauf angeordneten Blättern ist vorzugsweise in einer Umverpackung enthalten, die die Form eines Magazins oder Spender aufweisen kann.

Zur Verwendung kann dann so vorgegangen werden, dass man ein entrolltes oder entfaltetes Stück eines netzartigen Bahnmaterials mit den daraufliegenden getrockneten oder angefeuchteten Blättern in ein flaches Wasserbecken gleicher Größe einlegt. Die Blätter beginnen zu schwimmen und saugen sich mit Feuchtigkeit voll, wobei sie sich zu austauschaktiven Blättern rekonstituieren. Haben die Blätter die gewünschte Aktivität und Konsistenz erreicht, kann das am Behälterboden liegende Netz angehoben werden, und die erhaltene Verbundschicht kann direkt auf eine Körperoberfläche aufgelegt oder aufgewickelt werden. Es ist natürlich auch möglich, die Blätter einfach lose in ein entsprechendes Wasserbecken zu geben und mit Hilfe eines nachträglich eingeführten Netzes herauszufischen, so dass ein ähnliches Umschlagsmaterial erhalten wird.

Gemäß einer anderen Ausführungsform kann man die Blätter auch per Hand aus einem Einweichbecken fischen und auf ein bereitgehaltenes Netz auflegen und dann anschließend auf den Körper aufbringen.

Es ist jedoch auch möglich, durch Einweichen reaktivierte oder auch ggf. frischen Blätter direkt unter Bildung einer Blattschicht auf die Haut aufzulegen, beispielsweise als Abdeckung eines Externums.

Eine besondere Methode des Aufbringens einer Blattschicht kann auch darin bestehen, die Blätter auf eine mit Salzwasser vorbefeuchtete Haut aufzublasen. Dazu kann man so vorgehen, dass man ein Bahnmaterial mit einer freiliegenden Blattschicht vor einem Gebläse anordnet, das so ausgerichtet ist, dass die Blätter auf eine benachbarte, feuchte Körperoberfläche aufgeblasen werden und auf dieser kleben bleiben und auf diese Weise eine Schicht bilden. Es kann in diesem Zusammenhang eine besondere Vorrichtung verwendet werden, die eines der beschriebenen Vorprodukte verarbeitet, in dem die Vorrichtung eine Ausgabevorrichtung aufweist, aus der ein Bahnmaterial mit einer Blattschicht so abgezogen oder ausgeschoben werden kann, dass eine auf dem Bahnmaterial aufliegende Blattschicht freigelegt wird, das Bahnmaterial unter ein Gebläse gefördert oder ein Gebläse eingeschaltet wird, und der davon erzeugte Luftstrom nimmt die Blattschicht auf und überführt die Blätter auf die Körperoberfläche, die mit einem Körperumschlag versehen werden soll. Liegen die Blätter in dem Vorprodukt in getrockneter Form vor, kann die Vorrichtung auch mit einer zusätzlichen Befeuchtungsvorrichtung für die Blätter versehen sein, so dass diese die für ein gleichmäßiges Ankleben erforderliche Klebrigkeit und Elastizität gewinnen. Die endgültige Rekonstruktion kann auch auf einem vorher angefeuchteten Körper direkt erfolgen.

Die Einrichtung zum kontrollierten Befeuchten der Blätter ist beispielsweise eine Sprühvorrichtung, die einen Feuchtigkeitsnebel erzeugt, oder eine Befeuchtungswalze, beispielsweise eine Quetschwalze mit einem saugfähigen, z.B. schwammartigen Belag. Die Blätter können auch durch einen Flüssigkeitsvorhang geführt werden.

Nachfolgend wird die Erfindung anhand von Ausführungsbeispielen noch näher erläutert.

### Ausführungsbeispiel 1:

Zur Herstellung eines Körperumschlags wird die Haut eines Badegastes zuerst benetzt, beispielsweise unter Verwendung einer Dusche. Ein Pulver aus zerkleinertem Laminaria digitata (Fingertang) wird mit warmen Wasser angerührt. Dabei bildet sich ein Gel. Dieses wird auf die gewünschte Konsistenz mit warmen Wasser verdünnt und mit der Hand oder einem Applikator als Externum auf die Haut des Badegastes aufgetragen. Über diese Gelschicht werden frische Algenblätter, oder durch Einweichen getrockneter Algen Blätter erhaltene reaktivierte Algenblätter aufgelegt. Der behandelte Körperabschnitt kann kleinflächig oder großflächig sein, je nach Wunsch. Das Algenblatt befindet sich in dem gebildeten Umschlag in einem ähnlichen Milieu wie in der Natur. Es nimmt daher seine Austauschtätigkeit, die Teil seiner Lebensfunktionen ist, wieder auf.

Durch eine äußere Benetzung, beispielsweise einen Auftrag von Meerwasser, Salzlösungen (Sole) oder andere Flüssigkeiten, pastösen Medien, wie zum Beispiel Ölen, kann diese Austauschtätigkeit unterstützt, stabilisiert oder reguliert werden.

Auf die Blattschicht wird vorzugsweise noch eine dünne Okklusiv-Folie der erforderlichen Reinheit gelegt und angedrückt.

Der gebildete Okklusionsumschlag kann für die gewünschte Behandlungszeit auf dem Körper gelassen werden. Die beschriebene Behandlung kann von anderen Behandlungen begleitet werden, beispielsweise durch ein vorheriges oder anschließendes Aufbringen eines Öls oder einer Creme. Der Sauerstoffaustausch kann mechanisch, beispielsweise durch eine leichte Massage, weiter begünstigt werden.

### Ausführungsbeispiel 2:

Die Haut des Badegastes wird mit einer Sole oder mit Meerwasser oder einem anderen, die Herauslösung von Stoffen aus der Haut oder das Einbringen von Stoffen in die Haut fördernden flüssigen Medium benetzt. Auf den Flüssigkeitsfilm werden frische oder durch Einweichen reaktivierte Blätter einzeln, in Reihen oder in größeren Flächen aufgelegt. Der Stoffaustausch zwischen dem Flüssigkeitsfilm auf der Haut und der Haut selbst wird durch den einsetzenden aktiven Stoffaustausch in Richtung der aufgelegten Blätter aktiviert und reguliert. Je nach Zusammensetzung des Flüssigkeitsfilms, der Art der Blätter und der Behandlungszeit sowie unter Beachtung des Körperzustands des Patienten wird ein intensivierter Stoffaustausch erhalten, der zu Kur- und Heilzwecken genutzt werden kann.

Aufgrund der Möglichkeit, die erfindungsgemäß zu verwendenden Blätter (oder flächigen Blattteile) mit verschiedenen wirkstoffreichen, wirkstoffarmen oder wirkstofffreien, z.B. absorbierenden, Externaschichten zu kombinieren, können bei der erfindungsgemäßen Verwendung Körperumschläge mit z.B. folgendem Schichtaufbau erhalten werden:
1. Nasse Haut (als Grundlage) - Film aus einem brei- oder gelartig aufbereiteten Algenprodukt - Blätter - Folie;
2. Nasse Haut - Film aufbereiteter Algen - Blätter - eine weitere fördernde, stabilisierende oder retardierende Aufbringung, z.B. eine Sole - Folie;
3. Nasse Haut - eine fördernde, stabilisierende oder retardierende Aufbringung, z.B. eine Sole - Blätter - Film aufbereiteter Algen - Folie

## Patentansprüche

1. Verwendung von austauschaktiven Blättern von Tangen der Gruppen der Braunalgen, Rotalgen und Grünalgen in Form von ganzen Blättern oder von Blattteilen, bei denen wesentliche Flächenbereiche der Blattspreite ganzer Blätter erhalten sind und ein intensiver Stoffaustausch durch die Blattoberfläche erfolgt, in frischer Form oder in durch Einweichen getrockneter Blätter oder Blatteile reaktivierter Form zur Herstellung von Körperumschlägen für die kosmetische oder medizinisch-dermatologische Behandlung von Kurgästen und Patienten.

2. Verwendung nach Anspruch 1, **dadurch gekennzeichnet, daß** die Blätter - in Verbindung mit einem direkt auf die Haut aufzubringenden pastösen wirkstoffreichen Packungsmaterial oder einer Vehikelaufbringung - als Außenschicht von Okklusivumschlägen verwendet werden.

3. Verwendung nach Anspruch 1 oder 2, **dadurch gekennzeichnet, daß** die Blätter in Verbindung mit einer äußeren Kunststoff-Abdeckfolie als austauschfördernde Zwischenschicht von Okklusivumschlägen verwendet werden.

4. Verwendung nach einem der Ansprüche 1 bis 3, **dadurch gekennzeichnet, dass** die Blätter und/oder der Körperumschlag durch Lichteinwirkung aktiviert werden.

5. Vorprodukt zur Herstellung von Umschlägen unter Verwendung austauschaktiver Blätter nach einem der Ansprüche 1 bis 4, **dadurch gekennzeichnet, daß** es ein vlies-, gaze- oder netzartiges Bahnmaterial auf textiler Basis oder Papierbasis sowie darauf schichtartig ausgebreitete austauschaktive Blätter der Tange in trockner oder feuchter Form aufweist, und **dadurch gekennzeichnet, daß** das Vorprodukt vor dem Einsatz befeuchtet wird.

6. Vorprodukt nach Anspruch 5, **dadurch gekennzeichnet, daß** das Bahnmaterial als Rolle vorliegt, in der die Blattschichten so als Zwischenschichten vorhanden sind, daß beim Abziehen des Bahnmaterials von der Rolle ein mit einer Blattschicht belegtes Bahnmaterial erhalten wird.

7. Vorprodukt nach Anspruch 5, **dadurch gekennzeichnet, daß** das Bahnmaterial als Faltstapel aus einzelnen Abschnitten des Bahnmaterials vorliegt, in dem die Blattschichten so als Zwischenschichten vorhanden sind, daß beim Abziehen eines der Abschnitte von dem Faltstapel ein mit einer Blattschicht belegter Abschnitt Bahnmaterial erhalten wird.

8. Vorprodukt nach einem der Ansprüche 5 bis 7, **dadurch gekennzeichnet, daß** das Bahnmaterial in einer Umverpackung in Form eines Magazins oder Spenders enthalten ist.

9. Verwendung des Vorprodukts nach einem der Ansprüche 5 bis 8 zur Herstellung von Körperumschlägen für die kosmetische oder medizinisch-dermatologische Behandlung von Kurgästen und Patienten gemäß einem der Ansprüche 1 bis 4, **dadurch gekennzeichnet, daß** das Vorprodukt vor dem Einsatz befeuchtet wird.

10. Verwendung des Vorprodukts nach Anspruch 9, **dadurch gekennzeichnet, daß** das Vorprodukt mit Hilfe einer Sprühvorrichtung zur Erzeugung von Feuchtigkeitsnebel und/oder einer Befeuchtungswalze befeuchtet wird.

## Claims

1. Use of exchange-active leaves of seaweed of the groups of brown algae, red algae and green algae in the form of whole leaves or leaf parts, in which substantial surface areas of the leaf blade of whole leaves are obtained and an intensive substance exchange takes place through the leaf surface, in fresh form or in form reactivated by softening of dried leaves or leaf parts, to produce body wraps for the cosmetic or medicinal-dermatological treatment of spa guests and patients.

2. Use according to Claim 1, **characterised in that** the leaves, in connection with a paste-like packing material rich in active substances which is applied directly to the skin, or a vehicle application, is used as an outer layer of occlusive wraps.

3. Use according to Claim 1 or 2, **characterised in that** the leaves are used in connection with an outer plastic cover film as an exchange-promoting intermediate layer of occlusive wraps.

4. Use according to any one of Claims 1 to 3, **characterised in that** the leaves and/or the body wrap are activated by the effect of light.

5. Pre-product for production of wraps using exchange-active leaves according to any one of Claims 1 to 4, **characterised in that** it comprises a fleece, gauze or net-like web material on a textile basis or paper basis and spread thereon in a layer exchange-active leaves of seaweed in dry or moist form, and **characterised in that** the pre-product is moistened before use.

6. Pre-product according to Claim 5, **characterised in that** the web material is present as a roll in which the leaf layers are present as intermediate layers such that on extraction of the web material from the roll, a web material coated with a leaf layer is obtained.

7. Pre-product according to Claim 5, **characterised in that** the web material is present as a fold stack of individual sections of web material, in which the leaf layers are present as intermediate layers such that on extraction of one of the sections from the fold stack, a section of web material coated with a leaf layer is obtained.

8. Pre-product according to any one of Claims 5 to 7, **characterised in that** the web material is contained in an outer packing in the form of a magazine or dispenser.

9. Use of the pre-product according to any one of Claims 5 to 8 for production of body wraps for the cosmetic or medicinal-dermatological treatment of spa guests and patients according to any one of Claims 1 to 4, **characterised in that** the pre-product is moistened before use.

10. Use of the pre-product according to Claim 9, **characterised in that** the pre-product is moistened using a spray device to generate a moisture mist and/or using a wetting roller.

## Revendications

1. Utilisation de feuilles de fucus, actives sur le plan métabolique, parmi les groupes des algues brunes (phéophycées), algues rouges (rhodophycées) et algues vertes (chlorophycées) sous la forme de feuilles entières ou de parties de feuilles dans lesquelles sont contenues des zones de surface essentielles du limbe de feuilles entières et dans lesquelles s'effectue un étanche métabolique intense à travers la surface de feuille, sous forme fraîche ou sous forme réactivée par trempage de feuilles ou de parties de feuille séchées, en vue de produire des compresses corporelles pour le traitement cosmétique ou médical-dermatologique de curistes et de patients.

2. Utilisation selon la revendication 1, **caractérisée en ce que** les feuilles - en association avec un enveloppement riche en matière active, pâteux à appliquer directement sur la peau ou avec une déposition véhiculaire - sont utilisées à titre de couche extérieure de compresses occlusives.

3. Utilisation selon la revendication 1 ou 2, **caractérisée en ce que** les feuilles sont utilisées en association avec un film de couverture extérieur en matière plastique à titre de couche intermédiaire, favorisant les échanges, dans des compresses occlusives.

4. Utilisation selon l'une des revendications 1 à 3, **caractérisée en ce que** les feuilles et/ou la compresse corporelle sont activées par action de lumière.

5. Produit précurseur pour produire des compresses en utilisant des feuilles actives sur le plan métabolique selon l'une des revendications 1 à 4, **caractérisé en ce qu'**il comprend un matériau en bande du type nappe, gaze ou filet à base textile ou à base de papier, ainsi que des feuilles de fucus actives sur le plan métabolique, étalées en couches sur ledit matériau en bande et présentes sous forme sèche ou humide, et **caractérisé en ce que** le produit précurseur est humecté avant la mise en oeuvre

6. Produit précurseur selon la revendication 5, **caractérisé en ce que** le matériau en bande se présente sous forme de rouleau dans lequel les couches de feuilles se présentent comme des couches intermédiaires de telle sorte que lors d'enlèvement du matériau en bande depuis le rouleau, on obtient un matériau en bande garni d'une couche de feuille.

7. Produit précurseur selon la revendication 5, **caractérisé en ce que** le matériau en bande se présente sous forme de pile pliée constituée de tronçons individuels du matériau en bande, dans laquelle les couches de feuilles se présentent comme des couches intermédiaires de telle sorte que lors d'enlèvement de l'un des tronçons depuis la pile pliée, on obtient un tronçon de matériau en bande garni d'une couche de feuilles.

8. Produit précurseur selon l'une des revendications 5 à 7, **caractérisé en ce que** le matériau en bande est contenu dans un emballage extérieur sous la forme d'un magasin ou d'un distributeur.

9. Utilisation du produit précurseur selon l'une des revendications 5 à 8 pour produire des compresses corporelles pour le traitement cosmétique ou médical-dermatologique de curistes et de patients selon l'une des revendications 1 à 4, **caractérisée en ce que** le produit précurseur est humecté avant la mise en oeuvre.

10. Utilisation du produit précurseur selon la revendication 9, **caractérisée en ce que** le produit précurseur est humecté à l'aide d'un dispositif de pulvérisation pour produire un brouillard d'humidité et/ou d'un cylindre d'humectation.
